# EUROPEAN PATENT SPECIFICATION

(11) **EP 0 328 255 B1**
(45) Date of publication and mention of the grant of the patent: **22.09.1993**
(21) Application number: 89300476.2
(22) Date of filing: 19.01.1989
(51) Int. Cl.: A61K 37/66

(54) **A medicament for the treatment of a fibrotic disorder, and anti-fibrotic composition**
Medikament zur Behandlung von fibrotischer Störung und antifibrotische Verbindung
Médicament pour le traitement des troubles fibrotiques, et composition antifibrotique

(30) Priority: 25.01.1988 US 147973
(43) Date of publication of application: 16.08.1989
(73) Proprietor: BAKER CUMMINS DERMATOLOGICALS, INC., Miami, Florida 33178 (US)
(72) Inventor: Berman, Brian, Orinda, CA 94563 (CA); Duncan, Matthew R., 220, Concord, CA 94520 (CA)
(74) Representative: Lally, William

(56) References cited:
- EP-A- 0 180 737
- BIOLOGICAL ABSTRACTS, vol. 85, 1988, abstract 7776
- BIOLOGICAL ABSTRACT, vol. 84, 1987, abstract 1086
- BIOLOGICAL ABSTRACTS, vol. 84, 1987, abstract 28220
- CHEMICAL ABSTRACTS, vol. 107, no. 1, July 6, 1987, page 519, abstract 5512f
- THE JOURNAL OF BIOLOGICAL CHEMISTRY, vol. 262, no. 27, pages 13348-13351

## Description

The present invention relates the use of alpha- or beta-, human interferon in the manufacture of a medicament for the reversal of excessivly deposited connective tissue components caused by a fibrotic disorder in a tissue area.

Tissue fibrosis, characterized by excessive deposition of connective tissue components (most notably collagen) is the major pathological feature in various clinical conditions. The collagen deposition can take place in various internal organs, as in pulmonary fibrosis or liver cirrhosis. Skin is also commonly affected by fibrotic processes, and dermal fibrosis is the clinical pathological hallmark of several acquired and heritable cutaneous disorders.

In most cases, fibrosis is a reactive process, and several different factors can apparently modulate the pathways leading to tissue fibrosis. Such factors, largely elaborated by the inflammatory tissue reaction, include the local expansion of fibroblast subpopulations, immune modulation of the synthetic functions of fibroblasts, and altered regulation of various metabolic reactions governing the biosynthesis and degradation of the connective tissue components. Thus, the net accumulation of collagen in fibrosis is a result of imbalance between the factors leading to production and deposition or degradation and removal of collagen.

Although various modalities have been utilized to treat fibrotic diseases and disorders, none of these treatments have been particularly effective because they generally are directed to the symptoms of the disorders but not at the underlying pathology, namely, the imbalance in the metabolic factors regulating production, deposition, degradation and removal of collagen and other connective tissue components. Thus, for example, while topical corticosteroids have been used with some degree of success in treating the early, inflammatory stage of cutaneous keloid formation, such steroid therapy has little or no effect on the later, fibrotic stage when the keloids are actually formed as a result of excess collagen production.

It has been discovered in recent years, however, that activated T-lymphocytes and monocytes/macrophages can effectively modulate several fibroblast functions through the release of soluble macromolecular factors, collectively categorized as lymphokines (for the factors released by lymphocytes) and monokines (for the factors released by monocytes such as macrophages). Among the fibroblast functions modulated by these lymphokines and monokines is the production of fibrosis-forming collagen. See, e.g., Duncan, M.R., et al., J. Invest. Dermatol. , 83:377 (1984). In 1985, we identified gamma-interferon as the lymphokine and beta-interferon as the monokine responsible for inhibition of fibroblast collagen production as well as inhibition of late, but not early, fibroblast proliferation. Duncan, M.R. and Berman, B., J. Exp. Med., 162:516-27 (1985).

In a paper first published in full in 1987, we disclosed our later discovery that a reduced-collagen-producing phenotype in cultured scleroderma fibroblasts persisted after short term exposure to interferons, whether alpha-, beta- or gamma-. Duncan, M.R. and Berman, B., J. Clin. Invest., 79:1318-24 (1987). Notwithstanding that discovery, which was based solely on in vitro data, it was not at all clear whether interferons would be useful in the treatment of fibrotic tissue disorders in vivo, particularly disorders such as cutaneous keloid formation, scleroderma, progressive systemic sclerosis, and the like, where mere restoration of normal fibroblast collagen production levels may prevent further excess collagen deposition, but will not remove or degrade the fibrotic lesions already formed. Moreover, because of the complexity of the interacting metabolic factors relating to connective tissue matrix formation and degradation, the fact that interferons appeared to cause a persistent inhibition of fibroblast collagen production in vitro by no means proved that a similar effect would be observed in vivo.

Hence, notwithstanding the foregoing discoveries, there has been no method disclosed in the prior art for treating fibrotic tissue disorders in humans in a safe and effective manner to inhibit further fibrotic tissue formation and to reduce or remove entirely already-formed fibrotic lesions.

### SUMMARY OF THE INVENTION

There is disclosed the use of alpha- or beta-, human interferon in the manufacture of a medicament for the reversal of a fibrotic disorder in a tissue area.

In keeping with the foregoing object and others which will become apparent hereinafter, there is disclosed the local or systemic administration to a human patient suffering from a fibrotic tissue disorder of an amount of a human interferon sufficient to raise the total concentration of human interferons in the fibrotic tissue area from 10 to 4 x 10⁷ international units (IU) per millilitre.

According to the present invention there is provided the use of alpha-, or beta-, human interferon in the manufacture of a medicament for the reversal of a fibrotic disorder in a tissue area. In this respect, reference to alpha- or beta- human interferons is intended to encompass the sub-types thereon.

There is further disclosed an anti-fibrotic composition comprising human alpha- or beta-interferon or sub-unit thereof and a pharmaceutically acceptable carrier.

As will be further detailed below, the administration of human interferons by suitable methods and in suitable amounts sufficient to raise the concentration in the affected tissue area to the aforementioned concentration range serves the dual purposes of altering the phenotype of excess collagen-producing fibroblasts. as well as accelerating the enzymatic degradation of excess connective tissue matrix already deposited. The result, which has been clinically observed, is the inhibition of further fibrotic tissue formation and the removal of existing fibrotic tissue to return the affected area to a normal state.

### BRIEF DESCRIPTION OF THE DRAWINGS

FIG. 1 is a graph showing the variation over time in the lesional area of a keloid treated as specifically detailed in the Example set forth at the end of this specification.

FIG. 2 is a graph showing variation over time in the production of various connective tissue matrix factors by keloid fibroblasts before and after in vivo treatment expressed as a percentage of normal fibroblast production of those factors.

FIG. 3 is a table comparing the quantities of extracellular collagen derived from cultures of keloid fibroblasts, pre- and post-in vivo treatment, and normal, untreated fibroblasts. The table also compares collagen production of the same cells after in vitro treatment with interferon-alpha_{2b}.

FIG. 4 is a table comparing the quantities of glycosaminoglycans derived from the same in vitro cultures of fibroblasts as in FIG. 3.

FIG. 5 is a table comparing the quantities of fibronectin derived from the same in vitro cultures of fibroblasts as in FIG. 3.

FIG. 6 is a table comparing fibroblast proliferation times in the same in vitro cultures of fibroblasts as in FIG. 3.

FIG. 7 is a bar graph illustrating the comparative amounts of collagenase derived from in vitro cultures of keloid fibroblasts pre- and post-in vivo-treatment, and normal, untreated fibroblasts.

FIG. 8 is a table comparing the quantities of collagenase derived from the same in vitro cultures of fibroblasts as in FIG. 3.

### DETAILED DESCRIPTION OF THE INVENTION

Whereas we had previously disclosed that human interferons acted as persistent fibroblast deactivators, inhibiting both the growth and collagen production of normal fibroblasts and hypercollagen-producing fibroblasts, we have now discovered that human interferons also increase the collagenase activity of the fibroblasts, either by increasing collagenase production to normal levels in diseased fibroblasts or by removing some yet unknown collagenase inhibiting factor. This discovery led to the development of the disclosed use, which has been found effective in vivo in reversing human fibrotic disorders.

The novel method comprises the administration to a human patient suffering from a fibrotic tissue disorder of a sufficient amount of human interferon to raise the initial total concentration of human interferons in the affected tissue area to 10 to 4 x 10⁷ IU/ml. As used herein, "total concentration of human interferon" refers to the combined concentration, expressed in IU/ml, of all human interferons in the tissue area, whether, alpha-, beta- or gamma-interferons. In the case of dermal fibrosis, for example dermal keloids or scleroderma, the desired tissue concentration level may be achieved by injecting the human interferon intralesionally in sufficient quantity to achieve the necessary concentration, based on the size of the lesion. In the case of systemic fibrosis, intramuscular or intravenous routes of administration can be utilized. In the case of pulmonary fibrosis, the interferon can be incorporated into a solution to be administered intrabronchially by means of an inhalator.

In general, the specific method and route of administration selected is dependent on an assessment of the most efficient and quickest method to achieve the necessary inteferon concentration at the affected site. The disclosed use is not limited to any specific route of administration, and any method or route of administrations known to those skilled in the medical and pharmaceutical arts which achieves the desired tissue concentration of interferon is comprehended.

The human interferons utilized according to the invention may be naturally derived or recombinant DNA-derived human interferon. Alpha (leukocyte) or beta (fibroblast) interferons, and their subtypes, may be effectively used according to the invention.

In vitro data suggest that alpha and beta interferons, and their subtypes, may have more activity in vivo than gamma-interferon because of their greater effect on increasing fibroblast collagenase activity. Human recombinant DNA-derived alpha_{2b}-interferon has been shown particularly effective in vivo.

The interferon composition used may comprise one or more human interferons, whether of natural origin or recombinant DNA-derived, in any pharmaceutically acceptable vehicle suitable for the preferred route of administration to be utilized in treating a particular condition. Thus, if a parenteral route of administration is required, a suitable injectable solution of human interferons may be prepared by any conventional means known in the pharmaceutical arts. For example, such an injectable solution may compromise sterile isotonic saline, preservatives such as methylparaben or propylparaben, pH adjusters, and buffers. Similarly, any standard vehicle known in the art to be suitable for use in a vehicle for inhalation agents may be utilized to create a vehicle for the interferons to be administered as a nebulized spray from an inhalator, e.g., for treatment of pulmonary fibrosis. Suitable emulsifiers, dispersing agents and wetting agents may also be present.

A wide variety of suitable parenteral and inhalant vehicles are set forth in the standard text entitled "Remington's Pharmaceutical Sciences" (17ed. 1985). The invention is not limited to any particular vehicle or formulation for the human interferons administered. It is only necessary that the vehicle be compatible with human inteferons and one in which the interferons may be easily dissovled or dispersed.

The disclosed use is useful in reversing any fibrotic disorder in humans characterized by excessive fibroblast production of connective tissue matrix, including collagen, fibronectin and glycosaminoglycans (GAG). This includes, among others, the following disorders:
cutaneous keloid formation
progressive systemic sclerosis (PSS)
liver cirrhosis
idiopathic and pharmacologically induced pulmonary fibrosis
chronic graft versus-host disease
scleroderma (local and systemic)
Peyronie's disease
pharmacologically induced fibrosis of the penis
post-cystoscopic urethral stenosis
post-surgical internal adhesions
idiopathic and pharmacologically induced retroperitoneal fibrosis
myelofibrosis
To treat each of the above disorders, it is necessary only to administer a sufficient quantity of one or more human interferons, whether naturally derived or recombinant DNA-derived, to raise the total tissue concentration of human interferon in the affected area to 10 to 4 x 10⁷ IU/ml. A more preferred range of tissue concentrations of interferon is from 10³ to 10⁷ IU/ml. Administration of the interferons by any appropriate route and in any preferred vehicle may be repeated as often as deemed necessary to achieve the therapeutic goal of restoration of normal fibroblast function and degradation of fibrotic lesions. In each instance, however, the administration of interferon should raise the total tissue concentration of interferons in the affected area to the range specified above.

The dosage amounts of interferon administered to achieve the desired concentration in the affected tissue area will generally be higher when systemic routes of administration are utilized, as opposed to local administration, because upon sytemic administration the interferons will be diluted and metabolized to some degree before reaching the fibrotic site. The determination of dosage amounts of interferon on a case-by-case basis to achieve the desired tissue concentration levels is within the skill of those knowledgable in the medical and pharmaceutical arts.

As set forth in greater detail in the following Example, the disclosed use was found clinically effective in the reversal of a progressively enlarging cutaneous keloid. After only two intralesional injections of an interferon, the lesional area was reduced by 41%. Moreover, fibroblasts cultured from the keloid prior to and after interferon injection were compared with fibroblasts cultured from the patient's normal skin, revealing that while pre-interferon fibroblasts produced more collagen, more glycosaminoglycans and less collagenase than the patient's own normal fibroblasts, the post-interferon fibroblasts persistently produced normal or sub-normal amounts of collagen and glycosaminoglycans and exhibited normalized levels of collagenase activity. The result of the interferon treatment was, thus, not only cessation of fibrotic tissue production but reduction of the already existing fibrotic lesion through the normalization of collagenase.

The following Example provides a detailed illustration of the method for treating fibrotic disorders in humans.

### EXAMPLE

### Patient Treatment

A 55 year old man with a history of post-surgical cutaneous keloid formation developed a keloid in a site on his upper right arm 10 weeks after receiving 200 W/cm² as a test dose prior to planned CO₂ laser ablation of his cafe-au-lait macule. The untreated lesion was biopsied under 1% lidocaine local anesthesia before interferon treatment (pre-IFN) and then injected with 1.5 million IU IFN-alpha_{2b} in 0.15 ml of solution using a 30-gauge needle immediately and 5 days later. On the 9th day the keloid was biopsied (post-IFN) as was normal appearing skin in a corresponding anatomical site. The keloid was further injected with 1.5 million IU IFN-alpha_{2b} in 0.15 ml on days 31, 33, 37, 38 and 40. Lesional area was calculated gravimetrically from lesional outlines drawn on plastic.

### Interferons

The patient's keloid received intralesional injections (1.5 million IU/injection) of recombinant DNA-derived human IFN-alpha_{2b} (Intron A; Schering Corporation, Kenilworth, N.J.). Recombinant DNA-derived human IFN-alpha_{2b} (SCH 30500) used in the in vitro studies described herein was produced in Escherichia coli and supplied by the Schering Corporation, Kenilworth, N.J. This IFN_{2b} preparation (6.6 x 10⁷ IU/ml of phosphate buffered saline; specific activity of 1.2 x 10⁸ IU/mg protein) was added directly to fibroblast cultures after dilution to desired concentrations with Dulbecco's modified Eagle's medium (DME) containing 0.1% human serum albumin.

### Fibroblast Cultures

Primary fibroblast cultures were initiated from full thickness 2 mm punch biopsies using an explant method. The explants were cultured in 24 well microculture plates (2 cm² surface area; Linbro: Flow Labs, McLean VA) in DME containing 25 mM Hepes, 2mM glutamine, 100 IU/ml penicillin and 100 µg/ml streptomycin plus 20% heat-activated fetal calf serum (FCS) (Whittaker-MA Bioproducts, Walkersville, MA) at 37^{o} C in 5% CO₂ humidified atmosphere. These primary fibroblast cultures approached confluency after 36 to 42 days of growth and were subsequently trypsinized and subcultured in DME + 20% FCS with subpassage at weekly intervals. Fibroblasts from selected subcultures were then assayed for functional activity, namely, fibroblast growth, collagen production, glycosaminoglycan (GAG) production, fibronectin production and collagenase production.

### Clinical Response

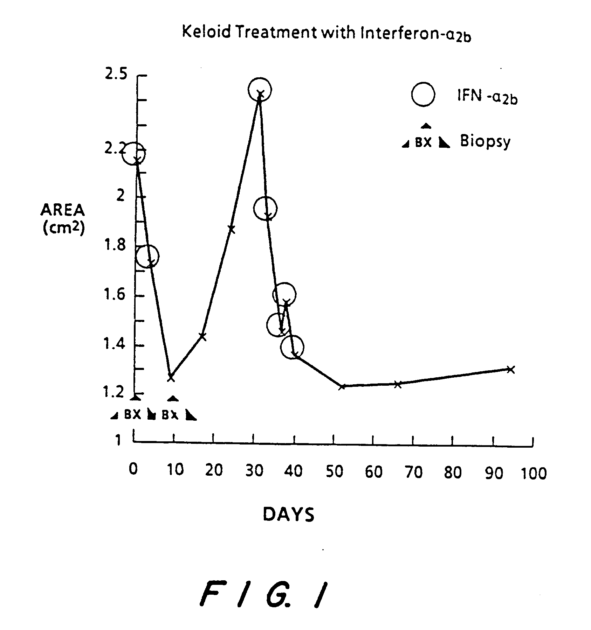

As shown in FIG. 1, the keloid injected intralesionally with IFN-alpha_{2b} on days 0 and 4 rapidly reduced in area by 41% by day 9 at which time a post-IFN bopsy of the keloid was performd. In light of the rapid increase in the area of the keloid which peaked on day 31, 5 additional IFN-alpha_{2b} doses were injected intralesionally during the subsequent 9 days. These treatments again resulted in a rapid, persistent reduction in lesional area (46% at day 94). Qualitatively, following each set of injections, the keloid became markedly softer and less raised. The patient experienced transient myalgias, which required no treatment, following the initial set of injections.

### In Vitro Production of Matrix Components By Keloidal and Normal Fibroblasts

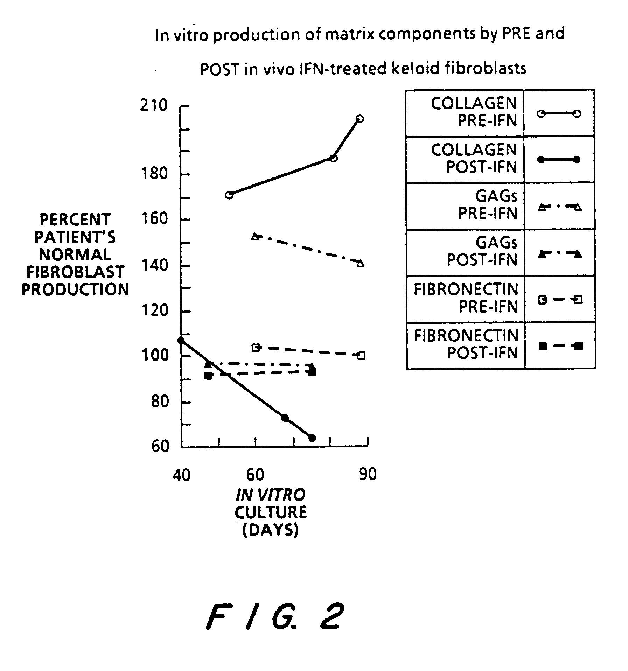

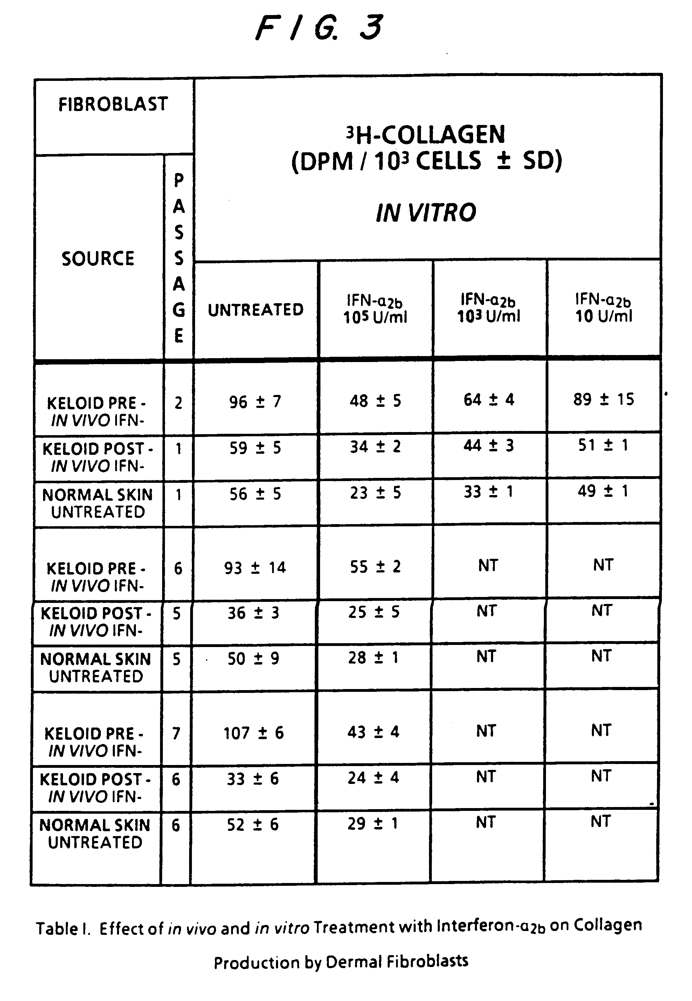

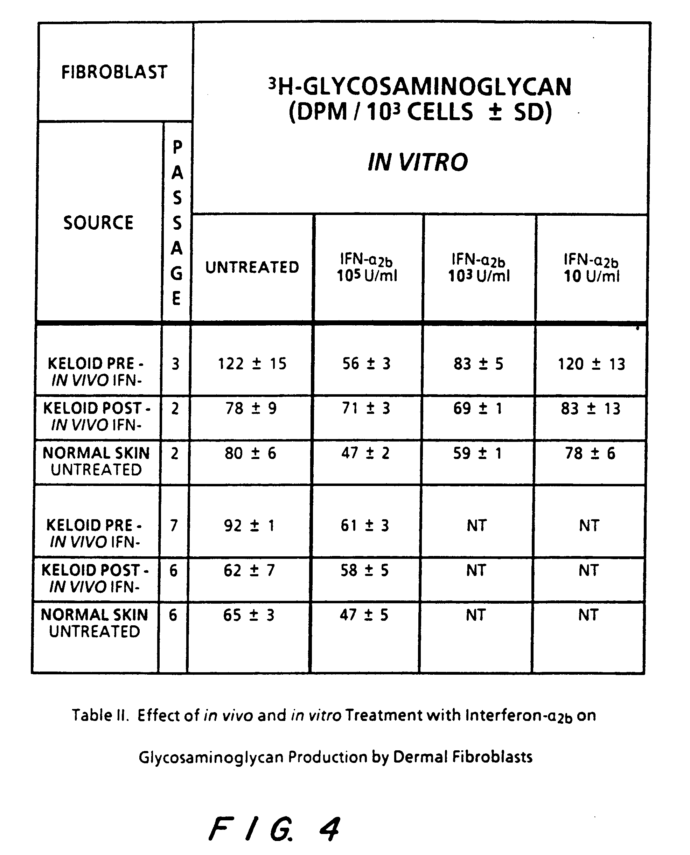

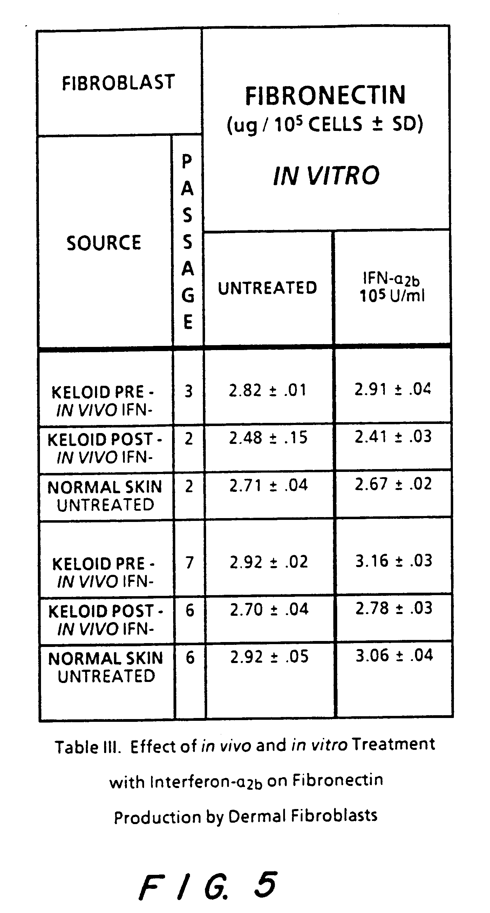

As summarized in FIG. 2, pre-IFN keloidal fibroblasts in vitro displayed an activated phenotype characterized by persistent hypercollagen and hyper-GAG production compared to the patient's normal fibroblasts. The initial set of IFN-alpha _{2b} injections in vivo resulted in a persistent de-activation of the keloidal fibroblast phenotype, with post-IFN keloidal fibroblasts producing normal or sub-normal amounts of collagen and GAGs. This persisten de-activation was selective, as the normal level of pre-IFN keloidal fibroblast production of fibronectin, another component of connective tissue matrix, remained normal in post-IFN keloidal fibroblasts. As detailed in Tables I and II (FIGS. 3 and 4), the addition of IFN-alpha_{2b} to confluent cultures of pre-IFN keloidal, post-IFN keloidal or normal fibroblasts resulted in a dose-related reduction in collagen and GAG production. Interestingly, a concentration range of 10³ - 10⁵ IU/ml, which theoretically is attainable locally in vivo, normalized the otherwise persistent hypercollagen and hyper-GAG production by pre-IFN keloidal fibroblasts. As shown in Table III (FIG. 5), in vitro exposure of different passages of any of the 3 fibroblast lines to IFN-alpha_{2b} (10⁵ IU/ml) failed to alter their normal level of firbonectin production.

### In Vitro Proliferation of Keloidal and Normal Fibroblasts

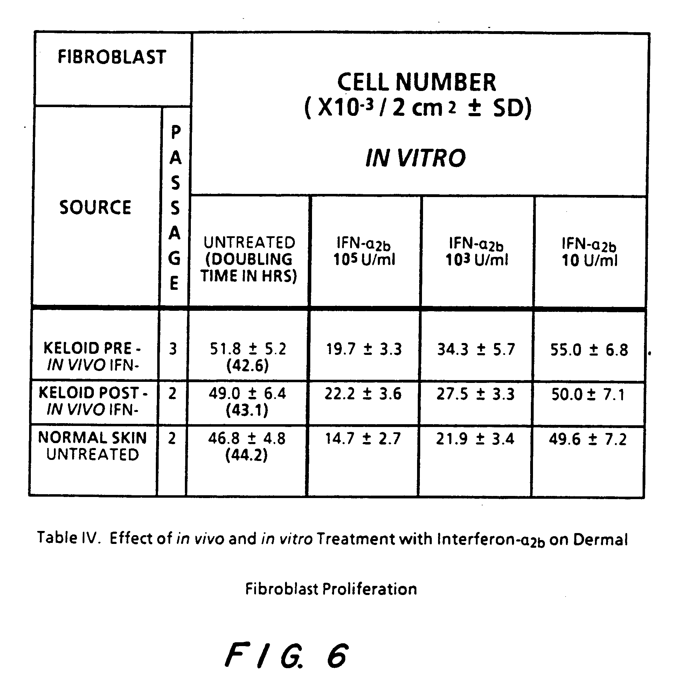

The observed effects of in vivo IFN-alpha_{2b} on in vitro functions of keloidal fibroblasts were not secondary to an anti-proliferative effect. Confluent non-proliferating cultures were examined, and as shown in Table IV (FIG. 6), in vivo exposure to IFN-alpha_{2b} failed to exert any lasting effect on proliferation, with the doubling time of pre-IFN, post-IFN and normal fibroblasts being virtually identical (p>0.5). It is likely that, similar to its effect in vitro (Table IV), exposure to IFN-alpha_{2b} inhibits fibroblast proliferation in vivo, but its continued presence may be required.

### In Vitro Elaboration of Collagenase Activity By Keloidal and Normal Fibroblasts

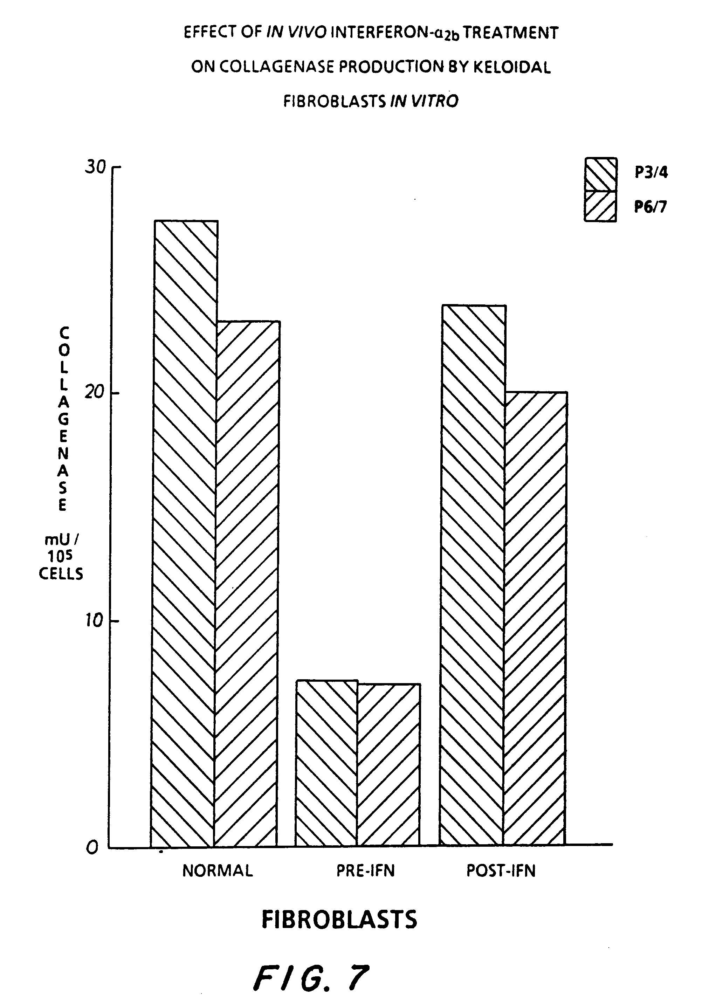

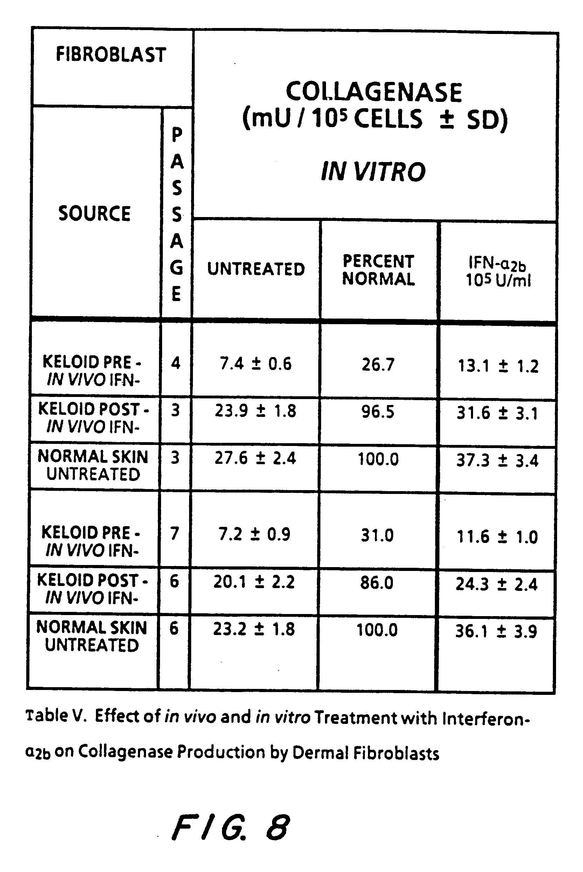

Although our detection of a persistent reduction of collagen and GAG production by keloidal fibroblasts exposed in vivo to IFN-a_{2b} could ultimately result in an antifibrotic effect, it was important to examine the potential catabolic activity of such fibroblasts on pre-formed collagen, especially in light of the rapid clinical response (FIG. 1). As shown in FIG. 7 keloidal fibroblasts elaborate less than 1/3 the normal amount of collagenase activity, which combined with their hyper-collagen and GAG production could result in the development of a keloid. Interestingly, in vivo exposure to IFN-alpha_{2b} normalized the collagenase activity elaborated by keloidal fibroblasts. This in vitro effect was persistent, being detected in early and late cell passages, in the absence of exogenous IFN. As shown in Table V (FIG. 8), in vitro exposure of each of the 3 fibroblast lines to IFN-alpha_{2b} (10⁵ IU/ml) increased the level of elaborated collagenase activity.

It has thus been shown that there are disclosed uses which achieve the various disclosed objects of the invention and which are well adapted to meet the conditions of practical use.

## Claims

1. The use of alpha- or beta-, human interferon in the manufacture of a medicament for the reversal of excessively deposited connective tissue components caused by a fibrotic disorder in a tissue area.

2. The use according to Claim 1 wherein the fibrotic disorder is selected from the group consisting of cutaneous keloid formation, progressive systemic sclerosis, liver cirrhosis, idiopathic and pharmacologically induced pulmonary fibrosis, chronic graft-versus-host disease, scleroderma (local and systemic), Peyronie's disease, pharmacologically induced fibrosis of the penis, post-cystoscopic urethral stenosis, post-surgical internal adhesions, myelofibrosis and idiopathic and pharmacologically induced retroperitoneal fibrosis.

3. The use according to Claim 2 wherein said fibrotic disorder is cutaneous keloid formation.

4. The use according to Claim 1, 2 or 3 wherein said interferon is alpha_{2b}-interferon.

5. The use according to any one of the preceding claims wherein said human interferon is naturally derived.

6. The use according to any one of the preceding claims wherein said interferon is recombinant DNA-derived.

7. Use according to any one of the preceding claims wherein the medicament is for reversal of excessivly deposited connective tissue components caused by a fibrotic disorder in a tissue area by administration of the medicament in a quantity sufficient to raise the total concentration of human interferons in the tissue area to between 10 and 4 x 10⁷, preferably between 10³ and 10⁷ international units per ml.

## Patentansprüche

1. Verwendung von menschlichem alpha- oder beta-Interferon zur Herstellung eines Arzneimittels zur Rückbildung von übermässig stark abgelagertem Bindegewebe-Komponenten, verursacht durch eine fibrotische Störung eines Gewebebereichs.

2. Verwendung nach Anspruch 1, worin die fibrotische Störung aus der Gruppe der Hautkeloidbildung, progressiver systemischer Sklerose, Leberzirrhose, idiopathischer und pharmakologisch ausgelöster Lungenfibrose, chronischer Implantat-Wirts-Krankheit, Skleroderma (örtlich und systemisch),Peyronie'sche Krankheit, pharmakologisch indizierte Fibrose des Penis, postcystokopischer Urethalstenose, postoperativen internen Anhaftungen, Myelofibrose und idiopathischer und pharmakologisch ausgelöster Retroperitonal-Fibrose ausgewählt ist.

3. Verwendung nach Anspruch 2, worin die fibrotische Störung Hautkeloidbildung ist.

4. Verwendung nach Anspruch 1, 2 oder 3, worin das genannte Interferon alpha_{2b}-Interferon ist.

5. Verwendung nach einem der vorstehenden Ansprüche, worin das genannte menschliche Interferon natürlichen Ursprungs ist.

6. Verwendung nach einem der vorstehenden Ansprüche, worin das genannte Interferon von rekombinanter DNA stammt.

7. Verwendung nach einem der vorstehenden Ansprüche, worin das Arzneimittel zur Rückbildung übermässig abgelagerter Bindegewebe-Komponenten, verursacht durch eine fibrotische Störung eines Gewebebereiches, durch Verabreichung des Arzneimittels in einer Menge bestimmt ist, die ausreicht, um die Gesamtkonzentration der menschlichen Interferone im Gewebebereich auf einen Wert zwischen 10 und 4 x 10⁷, vorzugsweise zwischen 10³ und 10⁷, internationalen Einheiten pro ml anzuheben.

## Revendications

1. Utilisation de l'interféron humain alpha ou bêta dans la préparation d'un médicament pour l'inversion d'un dépôt excessif de composants du tissu conjonctif, provoqué par un dérangement fibrotique dans une région de tissu.

2. Utilisation selon la revendication 1, dans laquelle le dérangement fibrotique est choisi dans le groupe constitué de formation de kéloïde cutané, de sclérose systémique progressif, de cirrhose de foie, de fibrose pulmonaire idiopathique et induit pharmacologiquement, d'une maladie chronique de greffe-contre-hôte, de sclérodermie (locale et systémique), de maladie de Peyronie, de fibrose du pénis induit pharmacologiquement, de sténose urétral post-cytoscopique, d'adhésions internes post-opératoires, de myélofibrose, et de fibrose rétropéritonéal induit pharmacologiquement.

3. Utilisation selon la revendication 2, dans laquelle le dérangement fibrotique est la formation de kéloïde cutané.

4. Utilisation selon la revendication 1, 2 ou 3, dans laquelle le dit interféron est l'alpha_{2b}-interféron.

5. Utilisation selon l'une quelconque des revendications précédentes, dans laquelle le dit interféron humaine est d'origine naturelle.

6. Utilisation selon l'une quelconque des revendications précédentes, dans laquelle le dit interféron est dérivé de ADN recombinant.

7. Utilisation selon l'une quelconque des revendications précédentes, dans laquelle le médicament est pour l'inversion d'un dépôt excessif de composants de tissu conjonctif causé par un dérangement fibrotique dans une région de tissu, par administration du médicament à raison d'une quantité suffisante pour élever la concentration totale d'interférons humains dans la région de tissu à une valeur située entre 10 et 4 x 10⁷, de préférence entre 10³ et 10⁷ unités internationales par ml.
